# EUROPEAN PATENT APPLICATION

(11) **EP 2 298 247 A1**
(43) Date of publication of application: **23.03.2011**
(21) Application number: 09171013.7
(22) Date of filing: 22.09.2009
(51) Int. Cl.: A61F 2/78, A61F 2/28, A61F 2/30

(54) **Osseointegration system for a long bone**

(71) Applicant: Rijksuniversiteit Groningen, 9712 CP Groningen (NL); Academisch Ziekenhuis Groningen, 9713 GZ Groningen (NL); Stichting Katholieke Universiteit, more particularly the Radboud University Nijmegen Medical Centre, 6500 HB Nijmegen (NL)
(72) Inventor: Tomaszewski, Pawel Krysztof, 9742 HN Groningen (NL); Verkerke, Gijsbertus Jacobus, 9756 TG Glimmen (NL); Verdonschot, Nicolaas Jacobus Joseph, 6524 WX Nijmegen (NL); Bulstra, Sjoerd Klaas, 9713 HM Groningen (NL)
(74) Representative: Hatzmann, Martin

(57) **Abstract**

An osseointegration system (1) for a long bone (2) comprises:
- a sleeve system comprising at least two osseointegrated sleeves (4, 5) being mutually uncoupled in the sense that respective ones of the sleeves do not prevent one another to perform transverse displacements relative to one another;
- a stem (6) arranged for being, in said operation condition, received within the at least two osseointegrated sleeves in such manner that at least low amplitude to-and-fro axial sliding displacements of the stem relative to said sleeves are allowed;
- at least one fitting collar (7); and
- retaining means (9) arranged for realizing that in said operation condition the stem is retained by the long bone.

## Description

The invention relates to an osseointegration system for a long bone.

The invention can be applied to any type of long bone, including the femurs, tibias and fibulas of the legs, the humeri, radii and ulnas of the arms, metacarpals and metatarsals of the hands and feet and the phalanges of the fingers and toes. The invention can be used in the field of prosthetic surgery, for example in case of hip, knee, ankle, shoulder, elbow or finger prostheses.

An osseointegration system for a distally amputated human femur is known from L. Zheng, J.M. Luo, B.C. Yang, J.Y. Chen, and X.D. Zhang, "3D Finite Element Analysis of Bone stress around Distally Osteointegrated Implant for Artificial Limb Attachment", Key Engineering Materials, Vols. 288-289 (2005), pp.653-656. Hereinafter, this prior art document is referred to as the "Zheng document".

The Zheng document discloses a multi-part implant, in particular for a distally truncated femur for prosthetic artificial lower limb attachment. The disclosed multi-part implant is composed of three components. A first one of these components is a stem 1 which has a long segment 1(B) having no contact with bone. The stem 1 also has a short, osseointegrated segment 1(A) of enlarged cross-sectional area relative to the cross-sectional area of the segment 1(B). This segment 1(A) is osseointegrated at the distal end of the truncated femur. Furthermore, the stem 1 has another short segment 1(C), which however has a decreased cross-sectional area relative to the cross-sectional area of segment 1(B). Segment 1(C) is located at an end of the stem 1 and is covered with a ringshaped second component 3 of the multi-part implant. This ringshaped component 3 is another osseointegrated part of the implant. The osseointegrated parts of the multi-part implant, i.e. the segment 1(A) and the ringshaped component 3, are positioned at the two opposite ends of the inserted part of the implant, with the long segment 1(B) (having no contact with bone) extending inbetween these two ends. The multi-part implant furthermore has a third component 2 being a silicon rubber circle serving as cushion to reduce impact.

The Zheng document discloses that, based on CT data and under the maximal load during a normal walking cycle, 3D finite element analysis was carried out to analyze the stress of bone around the multi-part implant in three cases of distally truncated femur at high-position, middle position and low-position. Results reveal that "stress shielding" (explained below) and stress concentration under the multi-part implant were not or hardly reduced compared with a traditional one-part implant, and that the stress concentration is redistributed.

It is remarked that the term "stress shielding", as used herein, refers to an undesirable reduction in bone density as a result of removal of normal stress from the bone by an implant. This is because bone in a person or animal will remodel in response to the loads that it experiences. Therefore, if the loading on a bone decreases, the bone will become less dense and weaker because there is no stimulus for continued remodeling that is required to maintain bone mass.

Note, that nonuniform stress distributions originating from implants may in the long term cause localised weakening of the bone, increasing the risks of bone fracture, other bone failures and implant loosening. Also note that implant loading generally is complex. It comprises mainly axial loads and bending loads, but also torsional loads, as well as simultaneously occurring combinations of such different loads. In addition, such complex loads may occur in the form of (heavy) impacts.

It is an object of the invention to at least provide an alternative osseointegration system, and, more in particular, to provide such an alternative osseointegration system which further improves uniformity of bone stress distribution under complex implant loadings, thus aiming at longer term preservation of bones with implants.

For that purpose, the invention provides an osseointegration system according to claim 1, while specific embodiments of the invention are set forth in the dependent claims.

Hence, the invention provides an osseointegration system for a long bone having an osteotomy end, the system comprising:
- a sleeve system comprising at least two sleeves, each of which is arranged for being, in operation condition, osseointegrated within the interior part of the long bone in such manner that the axial directions of the sleeves are extending in the longitudinal direction of the long bone with the sleeves being oriented substantially in line relative to one another and with the osseointegrated sleeves being mutually uncoupled in the sense that respective ones of the sleeves do not prevent one another to perform transverse displacements relative to one another;
- a stem arranged for being, in said operation condition, received within the at least two osseointegrated sleeves in such manner that at least low amplitude to-and-fro axial sliding displacements of the stem relative to said sleeves are allowed;
- at least one fitting collar arranged to protrude, in said operation condition, from said osteotomy end of the long bone, while the at least one fitting collar then is fitting onto said osteotomy end for transmitting axial loads from the osseointegration system to the long bone, and vice versa; and
- retaining means arranged for realizing that in said operation condition the stem is retained by the long bone.

Because the sleeves are mutually uncoupled in the said manner, a bending moment load on the stem will result in very slight transverse displacements of the respective sleeves relative to one another, each of the sleeves remaining substantially parallel to the longitudinal direction of the long bone during said displacements, while at the same time the mutually displaced sleeves each are transmitting the forces from the stem in a very uniformly distributed manner to the bone. Because said low amplitude to-and-fro axial sliding displacements of the stem relative to said sleeves are allowed and because axial loads are transmitted via the at least one fitting collar and via the osteotomy end, bending moment loads and axial loads are in fact decoupled from each other in the sense that via the circumferential walls of the sleeves the major part of the bending moment loads are transmitted, while via the at least one collar the major part of the axial loads are transmitted. This further contributes to a very uniform bone stress distribution, in particular under complex implant loadings, such as combined axial and bending moment loads. An additional function of the at least one fitting collar is sealing the medullary canal of the long bone so that germs are prevented from penetrating into the bone.

Preferably, the osseointegration system further comprising elastic material which, in said operation condition, is situated at least inbetween at least part of the sleeve system and at least part of the stem when the latter is thus being received within the at least two sleeves. The elastic material provides cushioning and promotes a uniform distribution of force transmittal between stem and sleeves and thus further contributes to a very uniform bone stress distribution. In addition, the presence of the elastic material further contributes to keeping each of the sleeves substantially parallel to the longitudinal direction of the long bone during the abovementioned very slight transverse displacements of the respective sleeves, originating from a bending moment load on the stem, and thus the presence of the elastic material further contributes to the abovementioned decoupling of bending moment loads and axial loads.

In a preferable embodiment, the at least two sleeves each have outside screw thread for being, in said operation condition, screwed down within the interior part of the long bone for being thus osseointegrated within the interior part of the long bone. Thus, the sleeves can be screwed down in a predrilled and/or pretapped hole in bone tissue. A coating on the outside (like hydroxy apatite) will stimulate bone ingrowth. However, instead of using outside screw thread, other means for osseointegrating the sleeves into the bone may be applied, for example in cases of sleeves which do not have a circular cross section and which therefore can not be screwed down in the bone. For example, various open-meshed, three-dimensional lattice structures may be applied, into which the connective tissue surrounding the sleeves after implantation grows in order to biologically fixate the device. Another option is applying V-shaped anti-removal grooves as known for orthopedic implants.

In a preferable embodiment, the stem comprises an integrated fixation collar, while said retaining means is arranged such that in said operation condition the integrated fixation collar is retained by the at least one fitting collar. The application of such an assembly of the integrated fixation collar and the at least one fitting collar allows for a highly uniform force transmission between the stem and said sleeve.

Preferably, the osseointegration system further comprises elastic material which, in said operation condition, is situated at least inbetween the at least one fitting collar and the integrated fixation collar when the at least one fitting collar is retaining the integrated fixation collar. The elastic material provides cushioning and also contributes to the realization of the low amplitude to-and-fro axial sliding displacements of the stem relative to the sleeves.

It is remarked that the at least one fitting collar may comprise a separate fitting collar. By the adjective "separate" used in the expression "separate fitting collar" it is meant that the separate fitting collar is neither an integrated part of a sleeve of the sleeve system nor an integrated part of the stem.

In a preferable embodiment, the at least one fitting collar comprises a first integrated fitting collar of a sleeve of the sleeve system, which first integrated fitting collar is arranged to protrude, in said operation condition, from said osteotomy end of the long bone, while the first integrated fitting collar then is fitting onto said osteotomy end for transmitting axial loads from the osseointegration system to the long bone, and vice versa. The application of such a first integrated fitting collar which is part of said sleeve contributes to strength and compactness of the osseointegration system.

In a preferable embodiment, the at least one fitting collar comprises a second integrated fitting collar of the stem, which second integrated fitting collar is arranged to protrude, in said operation condition, from said osteotomy end of the long bone, while the second integrated fitting collar then is fitting onto said osteotomy end for transmitting axial loads from the osseointegration system to the long bone, and vice versa. The application of such a second integrated fitting collar which is part of the stem contributes to strength and compactness of the osseointegration system. The second integrated fitting collar may be the only collar of the system to thus fit onto the osteotomy end. In that case, the second integrated fitting collar may for example be retained by a sleeve of the sleeve system and/or other parts of the stem may be retained by the sleeve system. Alternatively, however, it is possible that said second integrated fitting collar fits onto some parts of the osteotomy end, while another one of the at least one fitting collar fits onto other parts of the osteotomy end. In the lastmentioned case, the second integrated fitting collar and said other one of the at least one fitting collar may optionally be fastened to one another, in which case the second integrated fitting collar additionally performs the role of the abovementioned integrated fixation collar of the stem.

In a preferable embodiment, the at least one fitting collar which is then fitting onto said osteotomy end for transmitting axial loads from the osseointegration system to the long bone, and vice versa, comprises a non-flat exterior collar surface for matingly fitting, in said operation condition, onto a non-flat end surface of the osteotomy end for limiting relative tangential displacements between the osseointegration system and the long bone for transmitting torsional loads from the osseointegration system to the long bone, and vice versa. In this preferable embodiment the osteotomy end has to undergo a pre-treatment in order to receive its non-flat end surface. The application of such non-flat exterior collar surface and such non-flat end surface of the osteotomy end for transmitting torsional loads offers the advantage that thus the torsional loads are better transferred over the osteotomy end of the bone. Such non-flat surfaces may for example be cam surfaces, jagged surfaces, or the like.

In a preferable embodiment, the outside circumferential exterior surface of the stem and the inside circumferential exterior surface of at least one of the sleeves each have mutually mating shape provisions, such as mating key and key way provisions, arranged for limiting, in said operation condition, relative tangential displacements between the stem and the sleeve system for transmitting torsional loads from the osseointegration system to the long bone, and vice versa. The application of such mutually mating shape provisions of the stem and of the at least one of the sleeves offers the advantage that thus the transmitted torsional loads are uniformly distributed over the sleeve contacting areas of the bone.

In a preferable embodiment, the outside circumferential exterior surface of the stem and/or the inside circumferential exterior surface of at least one of the sleeves is/are shaped such that, in said operation condition, a radial distance between the outside circumferential exterior surface of the stem and the inside circumferential exterior surface of the concerned sleeve/sleeves is smaller at a mid-cross section of the concerned sleeve/sleeves than at an end-cross section of the concerned sleeve/sleeves. Such realization, in which said radial distance is smaller at said mid-cross section than at said end-cross section, further promotes that, in case of bending moment loads on the stem, the sleeves remain substantially parallel to the longitudinal direction of the long bone during the transverse displacements of the respective sleeves relative to one another, while at the same time the mutually displaced sleeves each are transmitting the forces from the stem in a very uniformly distributed manner to the bone.

In a preferable embodiment, a sleeve of the sleeve system has a closed end, which sleeve, relative to the other sleeve(s) of the sleeve system, is arranged for being osseointegrated within the interior part of the long bone the furthest away from the osteotomy end and with its closed end facing away from the osteotomy end. The application of such closed end prevents the marrow cavity of the bone, which marrow cavity is situated beyond said closed end on a side of the concerned sleeve facing away from the osteotomy end, to be pressurized by the low amplitude to-and-fro axial sliding displacements of the stem relative to the sleeves. Optionally, said marrow cavity may be filled up by an easily deformable material.

Further details, aspects and embodiments of the invention will be described, by way of example only, with reference to the schematic figure in the enclosed drawing.

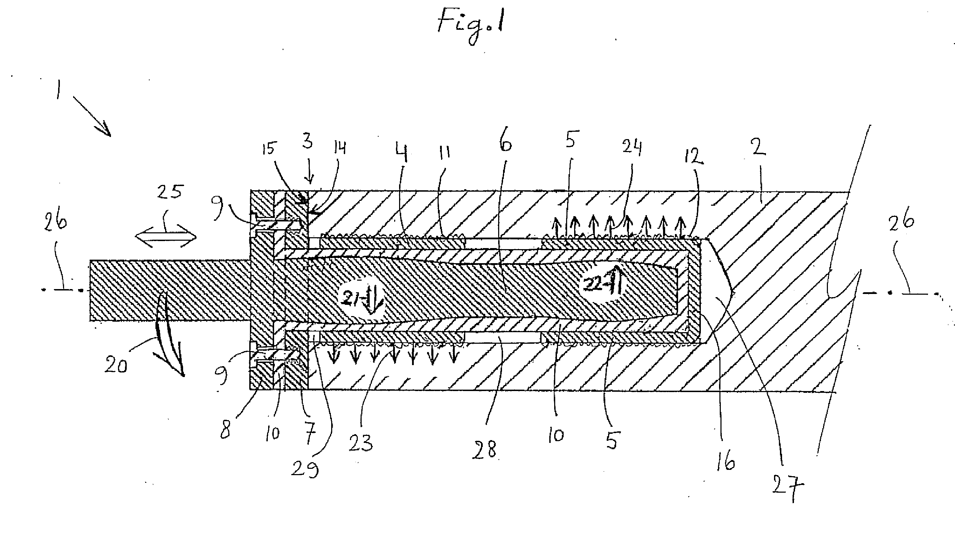

Fig. 1 shows, in longitudinal section, an example of an embodiment of an osseointegration system according to the invention.

In Fig. 1 there is shown the osseointegration system 1 in its operation condition in which it is osseointegrated with the long bone 2. The long bone 2 has an osteotomy end 3. In the shown example, the main configurations of the osseointegration system 1 and of the long bone 2 are substantially rotationally symmetrical relative to the longitudinal direction 26 of the long bone.

The osseointegration system 1 comprises a sleeve system which, in the shown example, comprises two sleeves 4 and 5. These two sleeves 4 and 5 each have outside screw thread 11 and 12, respectively, by which they have been screwed down within the interior part of the long bone 2 for thus being osseointegrated within the interior part of the long bone 2. The axial directions of the sleeves 4, 5 are extending in the longitudinal direction 26 of the long bone 2 with the sleeves 4, 5 being oriented substantially in line relative to one another. The osseointegrated sleeves 4, 5 are mutually uncoupled in the sense that they do not prevent one another to perform transverse displacements relative to one another.

The osseointegration system 1 further comprises a stem 6 which is received within the sleeves 4, 5 in such manner that at least low amplitude to-and-fro axial sliding displacements of the stem 6 relative to the sleeves 4, 5 are allowed. Inbetween the sleeves and the stem 6 there is elastic material 10.

It is remarked that Fig. 1 shows a space 28 of the long bone 2, which space 28 is located inbetween the sleeves 4 and 5. This space 28 may be filled up by elastic material 10. However, it is also possible that this space 28 is occupied by an additional (sleeve) element arranged for keeping the sleeves 4 and 5 at distance relative to one another. Also, it is possible that no such space 28 is present, for example in that the sleeves 4 and 5 are directly adjacent relative to one another.

The osseointegration system 1 further comprises a separate fitting collar 7 which protrudes from the osteotomy end 3 of the long bone 2 and which is fitting onto the osteotomy end 3 for transmitting axial loads from the osseointegration system 1 to the long bone 2, and vice versa. In Fig. 1 such axial loads are indicated by the double arrow 25. In the shown example, the separate fitting collar 7 is osseointegrated with the osteotomy end 3 of the long bone 2.

The stem 6 comprises an integrated fixation collar 8 which, by means of screws 9, is retained by the separate fitting collar 7. Thus, the stem 6 is retained by the long bone 2. Inbetween the separate fitting collar 7 and the integrated fixation collar 8 there is elastic material 10.

It is remarked that Fig. 1 shows a space 29 of the long bone 2, which space 29 is located inbetween the sleeve 4 and the separate fitting collar 7. This space 29 may be filled up by elastic material 10. However, it is also possible that this space 29 is occupied by an additional (sleeve) element arranged for keeping the sleeve 4 and the separate fitting collar 7 at distance relative to one another. Also, it is possible that no such space 29 is present, for example in that the sleeve 4 and the separate fitting collar 7 are directly adjacent relative to one another.

As mentioned, the osseointegrated sleeves 4, 5 are mutually uncoupled in the sense that they do not prevent one another to perform transverse displacements relative to one another. In Fig. 1 it is illustrated that a bending moment load 20 on the stem 6 will result in very slight transverse displacements 21, 22 of different sections of the stem 6 relative to the sleeves 4, 5. Because of the mentioned mutually uncoupled nature of the osseointegrated sleeves 4, 5, the bending moment load 20 on the stem 6 will also result in very slight transverse displacements of the respective sleeves 4, 5 relative to one another. These transverse displacements of the respective sleeves 4, 5 are in similar (mutually opposing) directions as the shown displacements 21, 22 of the stem 6. During their transverse displacements the sleeves 4, 5 will remain substantially parallel to the longitudinal direction 26 of the long bone 2. The mutually displaced sleeves 4, 5 each are transmitting the forces from the stem 6 in a very uniformly distributed manner to the bone. In Fig. 1, the lastmentioned uniform distributions of transmitted forces are indicated by two sets of arrows 23 and 24, respectively.

Because low amplitude to-and-fro axial sliding displacements of the stem 6 relative to the sleeves 4, 5 are allowed and because axial loads 25 are transmitted via the separate fitting collar 7 and via the osteotomy end 3, bending moment loads 20 and axial loads 25 are in fact decoupled from each other in the sense that via the circumferential walls of the sleeves 4, 5 the major part of the bending moment loads are transmitted, while via the separate fitting collar 7 the major part of the axial loads are transmitted. As mentioned, this results in a very uniform bone stress distribution, in particular under complex implant loadings, such as combined axial and bending moment loads.

It is remarked that in the shown example the enablement of the low amplitude to-and-fro axial sliding displacements of the stem 6, as well as of the very slight tilting displacements 21, 22 of the stem 6 is promoted by the presence of the elastic material 10 inbetween the fitting collar 7 and the fixation collar 8 and/or by the application, in a suitable manner, of some play in the fixation of the screws 9.

Although not shown in Fig. 1, the separate fitting collar 7 may comprise a non-flat exterior collar surface 14 for matingly fitting, in the shown operation condition, onto a non-flat end surface 15 of the osteotomy end 3 for limiting relative tangential displacements between the osseointegration system 1 and the long bone 2 for transmitting torsional loads from the osseointegration system to the long bone, and vice versa.

Although not shown in Fig. 1, the outside circumferential exterior surface of the stem 6 and the inside circumferential exterior surface of at least one of the sleeves 4, 5 each may have mutually mating shape provisions, such as mating key and key way provisions, arranged for limiting, in the shown operation condition, relative tangential displacements between the stem 6 and the sleeve system for transmitting torsional loads from the osseointegration system 1 to the long bone 2, and vice versa.

In Fig. 1 it is shown that the exterior circumferential surface of the stem 6 is shaped such that, in the shown operation condition, a radial distance between the exterior circumferential surface of the stem 6 and the interior circumferential surface of each of the sleeves 4, 5 is smaller at a mid-cross section of the concerned sleeve than at an end-cross section of the concerned sleeve. In the shown example, this is realized in that the cross section of the stem 6 at its axial locations corresponding to the locations of said mid-cross sections has a larger diameter than at its axial locations corresponding to the locations of said end-cross sections.

In the example of Fig. 1 it may be expedient when said radial distance, averaged over the axial range of the sleeve 5, is larger than said radial distance, averaged over the axial range of the sleeve 4. This in view of the fact that the absolute value of the tilting displacements 22, averaged over the axial range of the sleeve 5, will normally be larger than the absolute value of the tilting displacements 21, averaged over the axial range of the sleeve 4.

From Fig. 1 it can be seen that the sleeve 5, relative to the other sleeve 4 of the sleeve system, is arranged for being osseointegrated within the interior part of the long bone 2 the furthest away from the osteotomy end 3 of the sleeve system. In Fig. 1 it is shown that this sleeve 5 has a closed end 16 on its side facing away from the osteotomy end 3. The application of such closed end 16 prevents the marrow cavity 27 of the bone 2, which marrow cavity 27 is situated beyond said closed end 16 on a side of the sleeve 5 facing away from the osteotomy end 3, to be pressurized by the low amplitude to-and-fro axial sliding displacements of the stem 6 relative to the sleeves 4, 5. Optionally, the marrow cavity 27 may be filled up by an easily deformable material.

In the introduction of the present document it was described that, in a preferable embodiment, the at least one fitting collar comprises a first integrated fitting collar of a sleeve of the sleeve system, which first integrated fitting collar is arranged to protrude, in said operation condition, from said osteotomy end of the long bone, while the first integrated fitting collar then is fitting onto said osteotomy end for transmitting axial loads from the osseointegration system to the long bone, and vice versa. Such an embodiment is not shown in Fig. 1, but can be explained with reference to Fig. 1 by imagining in Fig. 1 that the space 29 is absent and that the collar 7 is an integrated collar of the sleeve 4.

In the introduction of the present document it was furthermore described that, in a preferable embodiment, the at least one fitting collar comprises a second integrated fitting collar of the stem, which second integrated fitting collar is arranged to protrude, in said operation condition, from said osteotomy end of the long bone, while the second integrated fitting collar then is fitting onto said osteotomy end for transmitting axial loads from the osseointegration system to the long bone, and vice versa. Such an embodiment is not shown in Fig. 1, but can be explained with reference to Fig. 1 by imagining in Fig. 1 that the collar 7 and the elastic material 10 inbetween the collar 7 and the collar 8 are absent, and that the collar 8 is fitting onto the osteotomy end 3 in such manner that the collar 8 performs said function of the second integrated fitting collar of the stem 6.

In the foregoing specification, the invention has been described with reference to specific examples of embodiments of the invention. It will, however, be evident that various modifications and changes may be made therein without departing from the broader scope of the invention as set forth in the appended claims.

For example, the number of the at least two sleeves of the sleeve system may be higher than two, such as three, four, five, six, seven, and so forth. Also, the perimeters of cross sections of the stem and of the sleeves may be noncircular. These perimeters may have various other forms, such as oval, polygonal, etcetera. Also, these perimeters may differ in dependence of the axial position along the sleeves and/or along the stem. Furthermore, the axial directions of the sleeves and/or of the stem and/or the longitudinal direction of the long bone do not necessarily have to be rectilinear. Instead, these axial and longitudinal directions may have various curved forms.

However, other modifications, variations and alternatives are also possible. The specifications and drawings are, accordingly, to be regarded in an illustrative rather than in a restrictive sense.

## Claims

1. Osseointegration system for a long bone (2) having an osteotomy end (3), the system (1) comprising:
- a sleeve system comprising at least two sleeves (4, 5), each of which is arranged for being, in operation condition, osseointegrated within the interior part of the long bone in such manner that the axial directions of the sleeves are extending in the longitudinal direction (26) of the long bone with the sleeves being oriented substantially in line relative to one another and with the osseointegrated sleeves (4, 5) being mutually uncoupled in the sense that respective ones of the sleeves do not prevent one another to perform transverse displacements relative to one another;
- a stem (6) arranged for being, in said operation condition, received within the at least two osseointegrated sleeves in such manner that at least low amplitude to-and-fro axial sliding displacements of the stem relative to said sleeves are allowed;
- at least one fitting collar (7) arranged to protrude, in said operation condition, from said osteotomy end (3) of the long bone (2), while the at least one fitting collar then is fitting onto said osteotomy end for transmitting axial loads from the osseointegration system to the long bone, and vice versa; and
- retaining means (9) arranged for realizing that in said operation condition the stem (6) is retained by the long bone (2).

2. Osseointegration system according to claim 1, further comprising elastic material (10) which, in said operation condition, is situated at least inbetween at least part of the sleeve system (4, 5) and at least part of the stem (6) when the latter is thus being received within the at least two sleeves.

3. Osseointegration system according to claim 1 or 2, wherein the at least two sleeves (4, 5) each have outside screw thread (11, 12) for being, in said operation condition, screwed down within the interior part of the long bone (2) for being thus osseointegrated within the interior part of the long bone.

4. Osseointegration system according to any one of the preceding claims, wherein the stem (6) comprises an integrated fixation collar (8), and wherein said retaining means (9) is arranged such that in said operation condition the integrated fixation collar (8) is retained by the at least one fitting collar (7).

5. Osseointegration system according to claim 4, further comprising elastic material (10) which, in said operation condition, is situated at least inbetween the at least one fitting collar (7) and the integrated fixation collar (8) when the at least one fitting collar is retaining the integrated fixation collar.

6. Osseointegration system according to any one of the preceding claims, wherein the at least one fitting collar comprises a first integrated fitting collar of a sleeve of the sleeve system, which first integrated fitting collar is arranged to protrude, in said operation condition, from said osteotomy end (3) of the long bone (2), while the first integrated fitting collar then is fitting onto said osteotomy end for transmitting axial loads from the osseointegration system to the long bone, and vice versa.

7. Osseointegration system according to any one of the preceding claims, wherein the at least one fitting collar comprises a second integrated fitting collar of the stem (6), which second integrated fitting collar is arranged to protrude, in said operation condition, from said osteotomy end (3) of the long bone (2), while the second integrated fitting collar then is fitting onto said osteotomy end for transmitting axial loads from the osseointegration system to the long bone, and vice versa.

8. Osseointegration system according to any one of the preceding claims, wherein the at least one fitting collar (7) which is then fitting onto said osteotomy end (3) for transmitting axial loads from the osseointegration system (1) to the long bone (2), and vice versa, comprises a non-flat exterior collar surface (14) for matingly fitting, in said operation condition, onto a non-flat end surface (15) of the osteotomy end (3) for limiting relative tangential displacements between the osseointegration system and the long bone for transmitting torsional loads from the osseointegration system to the long bone, and vice versa.

9. Osseointegration system according to any one of the preceding claims, wherein the outside circumferential exterior surface of the stem (6) and the inside circumferential exterior surface of at least one of the sleeves (4, 5) each have mutually mating shape provisions, such as mating key and key way provisions, arranged for limiting, in said operation condition, relative tangential displacements between the stem (6) and the sleeve system for transmitting torsional loads from the osseointegration system (1) to the long bone (2), and vice versa.

10. Osseointegration system according to any one of the preceding claims, wherein the outside circumferential exterior surface of the stem (6) and/or the inside circumferential exterior surface of at least one of the sleeves (4, 5) is/are shaped such that, in said operation condition, a radial distance between the outside circumferential exterior surface of the stem and the inside circumferential exterior surface of the concerned sleeve/sleeves is smaller at a mid-cross section of the concerned sleeve/sleeves than at an end-cross section of the concerned sleeve/sleeves.

11. Osseointegration system according to any one of the preceding claims, wherein a sleeve (5) of the sleeve system has a closed end (16), which sleeve (5), relative to the other sleeve(s) (4) of the sleeve system, is arranged for being osseointegrated within the interior part of the long bone (2) the furthest away from the osteotomy end (3) and with its closed end (16) facing away from the osteotomy end.
